(19) **Europäisches Patentamt**
**European Patent Office**
**Office européen des brevets**

(11) **EP 4 248 941 A1**

(12) **EUROPEAN PATENT APPLICATION**
published in accordance with Art. 153(4) EPC

(43) Date of publication:
**27.09.2023 Bulletin 2023/39**

(21) Application number: **21894743.0**

(22) Date of filing: **19.11.2021**

(51) International Patent Classification (IPC):
**A61K 8/58** (2006.01)   **A61K 8/06** (2006.01)
**A61K 8/88** (2006.01)   **A61K 8/895** (2006.01)
**A61Q 17/04** (2006.01)

(52) Cooperative Patent Classification (CPC):
**A61K 8/06; A61K 8/27; A61K 8/29; A61K 8/37;**
**A61K 8/58; A61K 8/81; A61K 8/88; A61K 8/891;**
**A61K 8/895; A61Q 17/04**

(86) International application number:
**PCT/JP2021/042525**

(87) International publication number:
**WO 2022/107872 (27.05.2022 Gazette 2022/21)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **19.11.2020   JP 2020192229**

(71) Applicant: **Kao Corporation**
**Chuo-ku**
**Tokyo 103-8210 (JP)**

(72) Inventors:
• **MATSUO, Mikano**
  **Odawara-shi, Kanagawa 250-0002 (JP)**
• **SOBU, Ryuutarou**
  **Odawara-shi, Kanagawa 250-0002 (JP)**

(74) Representative: **Hoffmann Eitle**
**Patent- und Rechtsanwälte PartmbB**
**Arabellastraße 30**
**81925 München (DE)**

(54) **OIL-IN-WATER EMULSIFIED COSMETIC**

(57) Provided is an oil-in-water type cosmetic composition which is excellent in an ultraviolet protection effect, an effect of suppressing the shine caused by sebum, and temporal stability. An oil-in-water type cosmetic composition comprising the following components (A), (B), (C), and (D):
(A) an ultraviolet absorber having an intramolecular siloxane bond;
(B) a highly polymerized silicone oil;
(C) a poly(meth)acrylamide compound; and
(D) a hydrophobized powder,
wherein a mass ratio of the component (B) to the component (A), [(B)/(A)], is 0.2 or more and 10 or less.

EP 4 248 941 A1

**Description**

Field of the Invention

The present invention relates to an oil-in-water type cosmetic composition.

Background of the Invention

In recent years, the importance of measures for suntan in daily life has been indicated, and oil-in-water sunscreen cosmetics which provide a fresh use impression and are easily used repeatedly have been developed. For example, Patent Literature 1 discloses an oil-in-water type cosmetic composition compounded with specific amounts of a specific solid organic ultraviolet absorber and a specific hydrophobized particulate metal oxide powder, and a polyacrylamide compound in addition to a liquid oil, which is considered to have a high ultraviolet protection effect, excellent temporal stability, and has less temporal viscosity change.

(Patent Literature 1) JP-A-2013-136569

Summary of the Invention

The present invention provides an oil-in-water type cosmetic composition comprising the following components (A), (B), (C), and (D):

(A) an ultraviolet absorber having an intramolecular siloxane bond;
(B) a highly polymerized silicone oil;
(C) a poly(meth)acrylamide compound; and
(D) a hydrophobized powder,

wherein a mass ratio of the component (B) to the component (A), [(B)/(A)], is 0.2 or more and 10 or less.

Detailed Description of the Invention

In the oil-in-water type cosmetic composition described in Patent Literature 1, an effect of suppressing the shine caused by sebum is insufficient.

The present invention provides an oil-in-water type cosmetic composition which is excellent in an ultraviolet protection effect, an effect of suppressing the shine caused by sebum, and temporal stability.

The present inventors found that an oil-in-water type cosmetic composition in which an ultraviolet absorber having an intramolecular siloxane bond and a highly polymerized silicone oil are combined in a specific mass ratio together with a poly(meth)acrylamide compound and a hydrophobized powder is excellent in the ultraviolet protection effect, the effect of suppressing the shine caused by sebum, and temporal stability, and has less stickiness after application, thereby completing the present invention.

The oil-in-water type cosmetic composition of the present invention is excellent in the ultraviolet protection effect, the effect of suppressing the shine caused by sebum, and temporal stability. It also has less stickiness after application.

<Component (A)>

The oil-in-water type cosmetic composition of the present invention comprises (A) an ultraviolet absorber having an intramolecular siloxane bond. Here, the component (B) highly polymerized silicone oil may separate in the oil phase and reduces the ultraviolet protection effect. However, in the present invention, the excellent ultraviolet protection effect can be imparted by coexistence of the component (A) together with the component (B) such that a mass ratio of [(B)/(A)] is 0.2 or more and 10 or less, in spite of the presence of the component (B) as described above.

The siloxane bond means a SiOSi bond, and examples of the ultraviolet absorber having an intramolecular siloxane bond include silicone-based ultraviolet absorbers and ultraviolet absorbers having a trisiloxane residue in the molecule.

As the silicone-based ultraviolet absorber, a compound in which a part of or all methyl groups in dimethicone are substituted with diethyl 4-propynyloxybenzalmalonate is preferable, and examples thereof include polysilicone-15 (dimethicodiethylbenzalmalonate) .

As the ultraviolet absorber having an intramolecular trisiloxane residue, an ultraviolet absorber having a trisiloxane residue and having a benzotriazole ring or a cinnamic acid ester skeleton in the molecule is preferable, and examples thereof include drometrizole trisiloxane and isopentyl trimethoxycinnamate trisiloxane.

Among these ultraviolet absorbers having an intramolecular siloxane bond, polysilicone-15 and drometrizole trisiloxane are preferable, from the viewpoint of the ultraviolet protection effect, the effect of suppressing the shine caused by sebum, and the like.

The ultraviolet absorbers having an intramolecular siloxane bond can be roughly classified into those in a solid state at 1 atm and 25°C, and those in a liquid state at 1 atm and 25°C. For example, the polysilicone-15 mentioned above is in a liquid state at 1 atm and 25°C. The drometrizole trisiloxane mentioned above is in a solid state at 1 atm and 25°C.

The ultraviolet absorber having an intramolecular siloxane bond may be used alone or in combination of two or more thereof.

The content of the ultraviolet absorber having an intramolecular siloxane bond is preferably 0.1% by mass or more, more preferably 0.5% by mass or more, further more preferably 0.7% by mass or more, further more preferably 1% by mass or more, and particularly preferably 1.3% by mass or more in the oil-in-water type cosmetic composition of the present invention, from the viewpoint of the ultraviolet protection effect, the effect of suppressing the shine caused by sebum, and the like, and is preferably 15% by mass or less, more preferably 10% by mass or less, further more preferably 5% by mass or less, and particularly preferably 4% by mass or less in the oil-in-water type cosmetic composition of the present invention, from the viewpoint of less stickiness after application and the like. The specific range is preferably 0.1% by mass or more and 15% by mass or less, more preferably 0.5% by mass or more and 10% by mass or less, further more preferably 0.7% by mass or more and 5% by mass or less, further more preferably 1% by mass or more and 4% by mass or less, and particularly preferably 1.3% by mass or more and 4% by mass or less in the oil-in-water type cosmetic composition of the present invention.

When the content of the component (A) is 0.7% by mass or more, 1% by mass or more, or 1.3% by mass or more, the ultraviolet protection effect and the effect of suppressing the shine caused by sebum are particularly improved. When the content of the component (A) is 5% by mass or less or 4% by mass or less, stickiness after application is particularly reduced.

<Component (B)>

The oil-in-water type cosmetic composition of the present invention comprises (B) a highly polymerized silicone oil. In the present invention, the (B) highly polymerized silicone oil of the component is a linear highly polymerized polymer having a segment consisting of repeating -SiO- as the main chain, and is a concept including those in which all of the side chains and terminals of polysiloxane are methyl groups and a part of the side chains is a phenyl group or hydrogen. The highly polymerized silicone oil of the component (B) is a concept excluding the component (A). By comprising the component (B), the shine caused by sebum can be suppressed.

As the highly polymerized silicone oil, a linear highly polymerized silicone oil being non-volatile liquid at 1 atm and 25°C is preferable.

The kinematic viscosity at 25°C of the highly polymerized silicone oil is preferably 8 $mm^2$/s or more, more preferably 10 $mm^2$/s or more, further more preferably 15 $mm^2$/s or more, further more preferably 20 $mm^2$/s or more, and particularly preferably 50 $mm^2$/s or more, from the viewpoint of the effect of suppressing the shine caused by sebum and the like, and is preferably 20 000 $mm^2$/s or less, more preferably 10 000 $mm^2$/s or less, further more preferably 7 500 $mm^2$/s or less, further more preferably 3 000 $mm^2$/s or less, further more preferably 1 000 $mm^2$/s or less, and particularly preferably 300 $mm^2$/s or less, from the viewpoint of less stickiness after application and the like. The specific range is preferably 8 $mm^2$/s or more and 10 000 $mm^2$/s or less, more preferably 10 $mm^2$/s or more and 7 500 $mm^2$/s or less, further more preferably 15 $mm^2$/s or more and 3 000 $mm^2$/s or less, further more preferably 20 $mm^2$/s or more and 1 000 $mm^2$/s or less, and particularly preferably 50 $mm^2$/s or more and 300 $mm^2$/s or less.

When the kinematic viscosity at 25°C of the highly polymerized silicone oil is 3 000 $mm^2$/s or less, 1 000 $mm^2$/s or less, or 300 $mm^2$/s or less, stickiness after application is particularly reduced.

The above kinematic viscosity at 25°C of the highly polymerized silicone oil is calculated using a capillary viscometer by measuring the time of a certain volume of liquid flowing through the capillary tube of the viscometer at 25°C and 1 atm and by using the following equation based on this outflow time and the viscometer constant. For example, an Ubbelohde or Cannon Fenske type capillary viscometer is typically used.

<Method for calculating kinematic viscosity>

$$\text{Kinematic viscosity (mm}^2\text{/s) = outflow time (second)} \times$$
$$\text{viscometer constant}$$

When two or more highly polymerized silicone oils are used, the kinematic viscosity of the mixture may fall within the above range.

Examples of the highly polymerized silicone oil include dimethicone, methylphenyl polysiloxane, and methylhydrogenpolysiloxane. Among these, dimethicone is preferable, from the viewpoint of the use impression, the effect of suppressing the shine caused by sebum, and the like.

Examples of commercial products of dimethicone include KF-96A-10CS (kinematic viscosity at 25°C: 10 mm$^2$/s), KF-96A-50CS (kinematic viscosity at 25°C: 50 mm$^2$/s), KF-96A-100CS (kinematic viscosity at 25°C: 100 mm$^2$/s), KF-96A-5000CS (kinematic viscosity at 25°C: 5 000 mm$^2$/s), and KF-96A-10000CS (kinematic viscosity at 25°C: 10 000 mm$^2$/s) (hereinabove, manufactured by Shin-Etsu Chemical Co., Ltd.). Examples of commercial products of methylphenyl polysiloxane include DOWSIL FZ-209 (manufactured by Dow Corning Toray Co., Ltd.).

The highly polymerized silicone oil may be used alone or in combination of two or more thereof.

The content of the highly polymerized silicone oil is preferably 0.1% by mass or more, more preferably 0.2% by mass or more, further more preferably 0.3% by mass or more, further more preferably 1.3% by mass or more, and particularly preferably 1.5% by mass or more in the oil-in-water type cosmetic composition of the present invention, from the viewpoint of the effect of suppressing the shine caused by sebum and the like, and is preferably 15% by mass or less, more preferably 10% by mass or less, further more preferably 7% by mass or less, and particularly preferably 5% by mass or less in the oil-in-water type cosmetic composition of the present invention, from the viewpoint of the ultraviolet protection effect, less stickiness after application, and the like. The specific range is preferably 0.1% by mass or more and 15% by mass or less, more preferably 0.3% by mass or more and 10% by mass or less, further more preferably 1.3% by mass or more and 7% by mass or less, and particularly preferably 1.5% by mass or more and 5% by mass or less in the oil-in-water type cosmetic composition of the present invention.

When the content of the component (B) is 1.3% by mass or more or 1.5% by mass or more, the effect of suppressing the shine caused by sebum is particularly improved. When the content of the component (B) is 7% by mass or less or 5% by mass or less, the ultraviolet protection effect and stickiness after application are particularly reduced.

A mass ratio of the component (B) to the component (A), [(B)/(A)], is 0.2 or more and 10 or less.

The mass ratio of [(B)/(A)] is preferably 0.4 or more, and more preferably 0.6 or more, from the viewpoint of the effect of suppressing the shine caused by sebum, less stickiness after application, and the like, and is preferably 5 or less, more preferably 3 or less, and particularly preferably 2 or less from the viewpoint of the ultraviolet protection effect, the effect of suppressing the shine caused by sebum, less stickiness after application, and the like. The specific range is preferably 0.4 or more and 5 or less, more preferably 0.4 or more and 3 or less, further more preferably 0.6 or more and 3 or less, and particularly preferably 0.6 or more and 2 or less.

When the mass ratio of [(B)/(A)] is 0.6 or more and 3 or less or 0.6 or more and 2 or less, the ultraviolet protection effect, the effect of suppressing the shine caused by sebum, and less stickiness after application are particularly excellent.

<Component (C)>

The oil-in-water type cosmetic composition of the present invention comprises (C) a poly(meth)acrylamide compound. The (meth)acrylamide is a generic term for acrylamide and methacrylamide.

The poly(meth)acrylamide compound may be a polymer having a repeating unit derived from (meth)acrylamide or a derivative thereof, and is a concept including polyacrylamide; polymethacrylamide; a poly(acrylamide derivative); a poly(methacrylamide derivative); a copolymer of (meth)acrylamide and a (meth)acrylamide derivative; and a copolymer of one or two or more monomers selected from the group consisting of acrylamide, methacrylamide, an acrylamide derivative, and a methacrylamide derivative and other monomers (e.g., (meth)acrylic acid, a (meth)acrylic acid salt, (meth)acrylate, and vinyl pyrrolidone). The poly(meth)acrylamide compound is preferably water soluble.

Here, the (meth)acrylamide derivative is preferably N-substituted (meth)acrylamide. Examples thereof include (meth)acryloyldimethyltaurine or a salt thereof. Examples of the salt of (meth)acryloyldimethyltaurine include alkali metal salts such as sodium salts, potassium salts, and lithium salts; salts with group 2 elements, such as calcium salts and magnesium salts; and ammonium salts.

The poly(meth)acrylamide compound is preferably a polymer having one or more repeating units selected from the group consisting of a repeating unit derived from (meth)acrylamide and a repeating unit derived from (meth)acryloyldimethyltaurine or a salt thereof, from the viewpoint of less stickiness after application, temporal stability, and the like. Examples thereof include poly(meth)acrylamide, a copolymer of hydroxyethyl (meth)acrylate and a (meth)acryloyldimethyltaurine salt, a copolymer of a (meth)acrylic acid salt and a (meth)acryloyldimethyltaurine salt, a copolymer of

**EP 4 248 941 A1**

(meth)acrylamide and a (meth)acrylic acid salt, a copolymer of (meth)acrylic acid, (meth)acrylamide, a (meth)acrylic acid salt, and a (meth)acryloyldimethyltaurine salt, and a copolymer of a (meth)acryloyldimethyltaurine salt and vinyl pyrrolidone.

Among these, poly(meth)acrylamide, a copolymer of hydroxyethyl (meth)acrylate and a (meth)acryloyldimethyltaurine salt, a copolymer of a (meth)acrylic acid salt and a (meth)acryloyldimethyltaurine salt, and a copolymer of a (meth)acryloyldimethyltaurine salt and vinyl pyrrolidone are preferable, from the viewpoint of less stickiness after application, temporal stability, and the like.

Examples of commercial products of polyacrylamide include SEPIGEL 305 (a mixture of polyacrylamide, hydrogenated polyisobutene, Laureth-7, and water).

Examples of commercial products of the copolymer of hydroxyethyl acrylate and an acryloyldimethyltaurine salt include SEPINOV EMT 10 (a mixture of a hydroxyethyl acrylate/sodium acryloyldimethyl taurate copolymer, sorbitan isostearate, and polysorbate 60), SIMULGEL NS (a mixture of a hydroxyethyl acrylate/sodium acryloyldimethyl taurate copolymer, squalane, polysorbate 60, and water), SIMULGEL FL (a mixture of a hydroxyethyl acrylate/sodium acryloyldimethyl taurate copolymer, isohexadecane, polysorbate 60, and water), and SEPIPLUS S (a mixture of a hydroxyethyl acrylate/sodium acryloyldimethyl taurate copolymer, polyisobutene, PEG-7 trimethylolpropane coconut ether, and water).

Examples of commercial products of the copolymer of an acrylic acid salt and an acryloyldimethyltaurine salt include SIMULGEL EG (a mixture of a sodium acrylate/sodium acryloyldimethyl taurate copolymer, isohexadecane, polysorbate 80, sorbitan oleate, and water).

Examples of commercial products of the copolymer of acrylamide and an acrylic acid salt include SEPIPLUS 265 (a mixture of an acrylamide/ammonium acrylate copolymer, polyisobutene, polysorbate 20, and water).

Examples of commercial products of the copolymer of an acrylic acid, acrylamide, an acrylic acid salt, and an acryloyldimethyltaurine salt include SEPIPLUS 400 (a mixture of polyacrylate-13, polyisobutene, polysorbate 20, and water).

Examples of commercial products of the copolymer of an acryloyldimethyltaurine salt and vinyl pyrrolidone include Aristoflex AVC (an ammonium acryloyldimethyltaurate/VP copolymer).

The poly(meth)acrylamide compound may be used alone or in combination of two or more thereof.

The content of the poly(meth)acrylamide compound is preferably 0.01% by mass or more, more preferably 0.05% by mass or more, further more preferably 0.1% by mass or more, and particularly preferably 0.3% by mass or more in the oil-in-water type cosmetic composition of the present invention, from the viewpoint of emulsion stability and the like, and is preferably 7.5% by mass or less, more preferably 5% by mass or less, further more preferably 2.5% by mass or less, and particularly preferably 2% by mass or less in the oil-in-water type cosmetic composition of the present invention, from the viewpoint of use impression and the like. The specific range is preferably 0.01% by mass or more and 7.5% by mass or less, more preferably 0.05% by mass or more and 5% by mass or less, further more preferably 0.1% by mass or more and 2.5% by mass or less, and particularly preferably 0.3% by mass or more and 2% by mass or less in the oil-in-water type cosmetic composition of the present invention.

A mass ratio of the component (A) to the component (C), [(A)/(C)] is preferably 0.1 or more, more preferably 0.5 or more, further more preferably 1 or more, and particularly preferably 2 or more, from the viewpoint of the ultraviolet protection effect, the effect of suppressing the shine caused by sebum, and the like, and is preferably 20 or less, more preferably 15 or less, further more preferably 10 or less, and particularly preferably 5 or less, from the viewpoint of less stickiness after application and the like. The specific range is preferably 0.1 or more and 20 or less, more preferably 0.5 or more and 15 or less, further more preferably 1 or more and 10 or less, and particularly preferably 2 or more and 5 or less.

A mass ratio of the component (B) to the component (C), [(B)/(C)] is preferably 0.1 or more, more preferably 1 or more, further more preferably 1.5 or more, and particularly preferably 2 or more, from the viewpoint of the effect of suppressing the shine caused by sebum and the like, and is preferably 20 or less, more preferably 15 or less, further more preferably 10 or less, and particularly preferably 7.5 or less, from the viewpoint of the ultraviolet protection effect, less stickiness after application, and the like. The specific range is preferably 0.1 or more and 20 or less, more preferably 1 or more and 15 or less, further more preferably 1.5 or more and 10 or less, and particularly preferably 2 or more and 7.5 or less.

<Component (D)>

The oil-in-water type cosmetic composition of the present invention comprises (D) a hydrophobized powder.

The hydrophobized powder is roughly classified into (D1) a hydrophobized ultraviolet scattering agent, and (D2) a hydrophobized powder other than the hydrophobized ultraviolet scattering agent. These may be used alone or in combination of two or more thereof. The shape of the hydrophobized powder is not particularly limited, and examples thereof include a spherical shape, a plate shape, a rod shape, a spindle shape, a needle shape, and an indefinite shape.

(Component (D1))

The hydrophobized ultraviolet scattering agent is preferably a hydrophobized particulate metal oxide.

The particulate metal oxide used for the hydrophobized particulate metal oxide is preferably one or more particulate metal oxides selected from the group consisting of zinc oxide, titanium oxide, cerium oxide, iron oxide, and chromium oxide, from the viewpoint of easy availability. Among these particulate metal oxides, one or more particulate metal oxides selected from the group consisting of zinc oxide, titanium oxide, and cerium oxide are preferable, and one or more particulate metal oxides selected from the group consisting of zinc oxide and titanium oxide are more preferable, from the viewpoint of the ultraviolet protection effect and the like. A trace element having +2 valent or higher can be incorporated into these particulate metal oxides, and one of metals such as iron, zirconium, calcium, manganese, magnesium, and yttrium, or a combination of two or more thereof can be incorporated into the particulate metal oxides.

The average primary particle size $d_A$ of the hydrophobized ultraviolet scattering agent is preferably 1 nm or more, more preferably 5 nm or more, and further more preferably 7 nm or more, and is preferably 100 nm or less, more preferably 80 nm or less, and further more preferably 50 nm or less, from the viewpoint of the ultraviolet protection effect and the like. The specific range of the average primary particle size $d_A$ is preferably 1 nm or more and 100 nm or less, more preferably 5 nm or more and 80 nm or less, and further preferably 7 nm or more and 50 nm or less.

The average primary particle size $d_A$ in the present specification can be determined from an observation image by a transmission electron microscope (TEM). Specifically, the average primary particle size $d_A$ can be determined by carrying out observation by TEM under the conditions of observation magnification: 50 000 times, measuring the maximum minor axis of 300 primary particles in the observation image, and calculating the number average value. Here, when the hydrophobized ultraviolet scattering agent has a shape other than the plate shape, the maximum minor axis refers to a minor axis having the maximum length among minor axes which orthogonally intersect major axes. When the hydrophobized ultraviolet scattering agent is the plate shape, the average primary particle size $d_A$ can be determined by measuring the thicknesses of 300 primary particles in an observation image observed under the same conditions as above, and calculating the number average value.

Examples of the hydrophobization treatment include silicone treatment such as methyl polysiloxane, methylphenyl polysiloxane, methylhydrogenpolysiloxane, methylcyclopolysiloxane, dodecamethylcyclohexasiloxane, tetradecamethylhexasiloxane, a dimethylsiloxane/methyl(polyoxyethylene)siloxane/methyl(p olyoxypropylene)siloxane copolymer, a dimethylsiloxane/methyl(polyoxyethylene)siloxane copolymer, a dimethylsiloxane/methyl(polyoxypropylene)siloxane copolymer, a dimethylsiloxane/methylcetyloxysiloxane copolymer, a dimethylsiloxane/methylstearoxysiloxane copolymer, and an acrylates/dimethicone copolymer; alkylalkoxysilane treatment such as octyl triethoxysilane and octyl trimethoxysilane; fatty acid treatment such as stearic acid, aluminium stearate, isostearic acid, and aluminium isostearate; fluorine-containing compound treatment such as perfluoroalkyl phosphate, perfluoroalcohol, and perfluoroalkyl alkoxysilane; amino acid treatment such as N-acylglutamic acid; alkyl phosphate treatment; and inorganic compound treatment such as ASI treatment. One of these treatments may be carried out, or a combination of two or more treatments may be carried out.

The treatment amount of the above surface treatment agent is preferably 1% by mass or more, and more preferably 3% by mass or more based on the total mass of the hydrophobized ultraviolet scattering agent, from the viewpoint of emulsion stability, dispersion stability, and the like, and is preferably 15% by mass or less, and more preferably 10% by mass or less based on the total mass of the hydrophobized ultraviolet scattering agent, from the viewpoint of emulsion stability, dispersion stability, and the like.

The above treatment amount means the amount when a total of the particulate metal oxide and the surface treatment agent is taken as 100% by mass. When the ultraviolet scattering agent has been subjected to hydrophobization treatment, the above average primary particle size $d_A$, the content, and the mass ratio in the cosmetic mean an average primary particle size $d_A$, content, and mass ratio including the surface treatment agent.

Examples of commercial products of hydrophobized particulate titanium oxide include MT-100 series, JR series, and MPY series manufactured by TAYCA CORPORATION, STR-100 series manufactured by Sakai Chemical Industry Co.,Ltd., and TTO55 series, TTO51 series, and MPT series manufactured by ISHIHARA SANGYO KAISHA, LTD. Examples of commercial products of hydrophobized particulate zinc oxide include MZ-500 series and MZ-300 series manufactured by TAYCA CORPORATION, and FINEX-50 series, FINEX-30 series, and FINEX-25 series manufactured by Sakai Chemical Industry Co., Ltd.

(Component (D2))

Examples of the hydrophobized powder other than the hydrophobized ultraviolet scattering agent include a powder obtained by subjecting an organic powder, an inorganic powder, or an organic-inorganic composite powder to hydrophobization treatment. Specific examples of the organic powder, inorganic powder, and organic-inorganic composite powder include metal oxides having an average primary particle size $d_A$ of more than 100 nm; pigments such as Red No. 104, Red No. 102, Red No. 226, Red No. 201, Red No. 202, Yellow No. 4, Black No. 401, and ultramarine; lake pigments such as Blue No. 1 aluminum lake, Yellow No. 4 aluminum lake, Yellow No. 5 aluminum lake, and Yellow No. 203 barium lake; polymers such as nylon powder, silk powder, urethane powder, Teflon (R) powder, silicone powder, polymethyl methacrylate powder, cellulose powder, and polyethylene powder; extender pigments such as talc, mica, sericite, kaolin, and flake-shaped barium sulfate; pearl pigments such as titanated mica, red iron oxide coated titanated mica, carmine coated titanated mica, iron blue coated titanated mica, and black iron oxide coated titanated mica; metal salts such as barium sulfate, calcium carbonate, magnesium carbonate, aluminum silicate, and magnesium silicate; inorganic powder such as silica, alumina, and red iron oxide; bentonite; smectite; boron nitride; and lauroyl lysine.

Examples of the hydrophobization treatment for the powder of the component (D2) include the same hydrophobization treatment as for the ultraviolet scattering agent of the component (D1).

As the metal oxide having an average primary particle size $d_A$ of more than 100 nm, one or more metal oxides selected from the group consisting of zinc oxide, titanium oxide, cerium oxide, iron oxide, and chromium oxide are preferable, from the viewpoint of easy availability.

The average primary particle size $d_A$ of the metal oxide used for the component (D2) is more than 100 nm, preferably from 150 to 500 nm, and more preferably from 200 to 400 nm.

The average primary particle size $d_A$ of the metal oxide used for the component (D2) may be measured in the same manner as the average primary particle size $d_A$ of the hydrophobized ultraviolet scattering agent.

The hydrophobized powder may be used alone or in combination of two or more thereof.

The content of the hydrophobized powder is preferably 1% by mass or more, more preferably 2% by mass or more, further more preferably 3% by mass or more, and particularly preferably 5% by mass or more in the oil-in-water type cosmetic composition of the present invention, from the viewpoint of the ultraviolet protection effect and the like, and is preferably 35% by mass or less, more preferably 30% by mass or less, further more preferably 20% by mass or less, and particularly preferably 17% by mass or less in the oil-in-water type cosmetic composition of the present invention, from the viewpoint of less stickiness after application, less friction feeling during application, temporal stability, and the like. The specific range is preferably 1% by mass or more and 35% by mass or less, more preferably 2% by mass or more and 30% by mass or less, further more preferably 3% by mass or more and 20% by mass or less, and particularly preferably 5% by mass or more and 17% by mass or less in the oil-in-water type cosmetic composition of the present invention.

When the content of the hydrophobized powder is 20% by mass or less or 17% by mass or less, stickiness after application and friction feeling during application are particularly reduced.

When the hydrophobized ultraviolet scattering agent is used as the hydrophobized powder, the content of the hydrophobized ultraviolet scattering agent is preferably 1% by mass or more, more preferably 2% by mass or more, and further more preferably 4% by mass or more in the oil-in-water type cosmetic composition of the present invention, from the viewpoint of the ultraviolet protection effect and the like, and is preferably 25% by mass or less, more preferably 20% by mass or less, and further more preferably 15% by mass or less in the oil-in-water type cosmetic composition of the present invention, from the viewpoint of less stickiness after application, less friction feeling during application, temporal stability, and the like. The specific range is preferably 1% by mass or more and 25% by mass or less, more preferably 2% by mass or more and 20% by mass or less, and further preferably 4% by mass or more and 15% by mass or less in the oil-in-water type cosmetic composition of the present invention.

The content of the hydrophobized powder other than the hydrophobized ultraviolet scattering agent is preferably 0% by mass or more, and more preferably 0.5% by mass or more in the oil-in-water type cosmetic composition of the present invention, and is preferably 12% by mass or less, and more preferably 8% by mass or less in the oil-in-water type cosmetic composition of the present invention, from the viewpoint of less friction feeling during application and the like. The specific range is preferably 0% by mass or more and 12% by mass or less, and more preferably 0.5% by mass or more and 8% by mass or less in the oil-in-water type cosmetic composition of the present invention.

A mass ratio of the component (D) to the component (B), [(D)/(B)] is preferably 0.1 or more, more preferably 0.5 or more, further more preferably 1 or more, and particularly preferably 2 or more, from the viewpoint of the ultraviolet protection effect, less stickiness after application, and the like, and is preferably 20 or less, more preferably 15 or less, further more preferably 13 or less, and particularly preferably 10 or less from the viewpoint of less friction feeling during application, the effect of suppressing the shine caused by sebum, and the like. The specific range is preferably 0.1 or more and 20 or less, more preferably 0.5 or more and 15 or less, further more preferably 1 or more and 13 or less, and particularly preferably 2 or more and 10 or less.

<Component (E)>

The content of (E) water is preferably 15% by mass or more, more preferably 20% by mass or more, further more preferably 25% by mass or more, and particularly preferably 30% by mass or more in the oil-in-water type cosmetic composition of the present invention, from the viewpoint of use impression and the like, and is preferably 80% by mass or less, more preferably 70% by mass or less, further more preferably 65% by mass or less, and particularly preferably 60% by mass or less in the oil-in-water type cosmetic composition of the present invention, from the viewpoint of temporal stability and the like. The specific range is preferably 15% by mass or more and 80% by mass or less, more preferably 20% by mass or more and 70% by mass or less, further more preferably 25% by mass or more and 65% by mass or less, and particularly preferably 30% by mass or more and 60% by mass or less in the oil-in-water type cosmetic composition of the present invention.

In addition to each component described above, the oil-in-water type cosmetic composition of the present invention preferably further comprises one or more selected from the group consisting of (F) an ultraviolet absorber other than the component (A), (G) a liquid oil agent (the liquid oil agent as the component (G) is a concept excluding organic ultraviolet absorbers being liquid at 1 atm and 25°C and the component (B), and hereinafter, the liquid oil agent as the component (G) is also referred to as "other liquid oil agents"), and (H) a surfactant, from the viewpoint of the ultraviolet protection effect and the like.

<Component (F)>

The ultraviolet absorber other than the component (A) is preferably an organic ultraviolet absorber other than the component (A).

Examples of the organic ultraviolet absorber other than the component (A) include benzoic acid-based ultraviolet absorbers, anthranilic acid-based ultraviolet absorbers, salicylic acid-based ultraviolet absorbers, cinnamic acid-based ultraviolet absorbers, benzophenone-based ultraviolet absorbers, and triazine-based ultraviolet absorbers. Among these, one or more selected from the group consisting of benzoic acid-based ultraviolet absorbers and triazine-based ultraviolet absorbers are preferable, from the viewpoint of the ultraviolet protection effect, less stickiness after application, temporal stability, and the like. As the organic ultraviolet absorber, an oil-soluble organic ultraviolet absorber is preferable.

Examples of the benzoic acid-based ultraviolet absorber include para-aminobenzoic acid (PABA), glyceryl PABA, ethyldihydroxypropyl PABA, N-ethoxylate PABA ethyl ester, N-dimethyl PABA ethyl ester, N-dimethyl PABA butyl ester, N-dimethyl PABA amyl ester, octyldimethyl PABA, and diethylamino hydroxybenzoyl hexyl benzoate.

Examples of the anthranilic acid-based ultraviolet absorber include homomenthyl-N-acetyl anthranilate.

Examples of the salicylic acid-based ultraviolet absorber include amyl salicylate, menthyl salicylate, homomenthyl salicylate, octyl salicylate, phenyl salicylate, benzyl salicylate, and p-isopropanol phenyl salicylate.

Examples of the cinnamic acid-based ultraviolet absorber include octyl cinnamate, ethyl-4-isopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, 2-ethylhexyl-p-methoxycinnamate, 2-ethoxyethyl-p-methoxycinnamate, cyclohexyl-p-methoxycinnamate, ethyl-$\alpha$-cyano-$\beta$-phenylcinnamate, 2-ethylhexyl-$\alpha$-cyano-$\beta$-phenylcinnamate, and glycerylmono-2-ethylhexanoyl diparamethoxycinnamate.

Examples of the benzophenone-based ultraviolet absorber include 2,4-dihydroxybenzophenone, 2,2'-dihydroxy-4-methoxybenzophenone, 2,2'-dihydroxy-4,4'-dihydroxybenzophenone, 2-hydroxy-4-methoxybenzophenone, 2-hydroxy-4-methoxy-4'-methylbenzophenone, 2-hydroxy-4-methoxybenzophenone, 4-phenylbenzophenone, 2-ethylhexyl-4'-phenylbenzophenone-2-carboxylate, 2-hydroxy-4-n-octoxybenzophenone, and 4-hydroxy-3-carboxybenzophenone.

Examples of the triazine-based ultraviolet absorber include 2,4,6-tris[4-(2-ethylhexyloxycarbonyl)anilino]-1,3,5-triazine, dioctyl butamido triazone, and 2,4-bis-[{4-(2-ethylhexyloxy)-2-hydroxy}-phenyl]-6-(4-methoxyphenyl)-1,3,5-triazine.

Examples of other organic ultraviolet absorbers include 3-(4'-methylbenzylidene)-dl-camphor, 3-benzylidene-dl-camphor, ethyl urocanate ester, 2-phenyl-5-methylbenzoxazole, 2,2'-hydroxy-5-methylphenylbenzotriazole, 2-(2'-hydroxy-5-t-octylphenyl)benzotriazole, dibenzalazine, dianisoylmethane, 4-methoxy-4'-t-butyldibenzoylmethane, 5-(3,3-dimethyl-2-norbonylidene)-3-pentane-2-one, a benzene-bis-1,3-diketone derivative described in JP-A-H2-212579, and a benzoylpinacolone derivative described in JP-A-H3-220153.

The organic ultraviolet absorber other than the component (A) can be classified into organic ultraviolet absorbers in a solid state at 1 atm and 25°C and organic ultraviolet absorbers in a liquid state at 1 atm and 25°C. Among these, organic ultraviolet absorbers in a solid state at 1 atm and 25°C are preferable, from the viewpoint of the ultraviolet protection effect, less stickiness after application, temporal stability, and the like. For example, diethylamino hydroxybenzoyl hexyl benzoate, 2,4-bis-[{4-(2-ethylhexyloxy)-2-hydroxy}-phenyl]-6-(4-methoxyphenyl)-1,3,5-triazine, 2,4,6-tris[4-(2-ethylhexyloxycarbonyl)anilino]-1,3,5-triazine, and the like mentioned above are the organic ultraviolet absorbers in a solid state at 1 atm and 25°C. Isopropyl-p-methoxycinnamate, 2-ethylhexyl-p-methoxycinnamate, 2-ethoxyethyl-p-methoxycinnamate, and the like mentioned above are the organic ultraviolet absorbers in a liquid state at 1 atm and 25°C.

The ultraviolet absorber other than the component (A) may be used alone or in combination of two or more thereof.

The content of the ultraviolet absorber other than the component (A) is preferably 0% by mass or more, more preferably 0.5% by mass or more, and further more preferably 2% by mass or more in the oil-in-water type cosmetic composition of the present invention, from the viewpoint of the ultraviolet protection effect and the like, and is preferably 15% by mass or less, more preferably 10% by mass or less, and further more preferably 7.5% by mass or less in the oil-in-water type cosmetic composition of the present invention from the viewpoint of use impression (in particular, less stickiness after application), temporal stability, and the like. The specific range is preferably 0% by mass or more and 15% by mass or less, more preferably 0.5% by mass or more and 10% by mass or less, and further preferably 2% by mass or more and 7.5% by mass or less in the oil-in-water type cosmetic composition of the present invention.

<Component (G)>

Other liquid oil agents refer to oil agents excluding organic ultraviolet absorbers being liquid at 1 atm and 25°C and the component (B) among oil agents in a liquid state at 1 atm and 25°C, and may be volatile oil agents or non-volatile oil agents. Other liquid oil agents may be used alone or in combination of two or more thereof. The volatile oil agent exhibits volatility at 25°C, and the non-volatile oil agent does not exhibit volatility at 25°C.

Examples of the volatile oil agent include light isoparaffin, decamethylcyclopentasiloxane, octamethylcyclotetrasiloxane, dodecamethylcyclohexasiloxane, methyl trimethicone, decamethyltetrasiloxane, ethyl trisiloxane, and volatile methylpolysiloxane.

Examples of commercial products of the light isoparaffin include Isopar H (manufactured by Esso Chemical Limited), isododecane (manufactured by Bayer AG), isohexadecane (manufactured by Uniqema), and IP SOLVENT 1620MU, IP SOLVENT 2028MU, and IP SOLVENT 2835 (hereinabove, manufactured by Idemitsu Kosan Co., Ltd.). Examples of commercial products of the decamethylcyclopentasiloxane include TFS405 (manufactured by Momentive Performance Materials Japan LLC.), SH245 and DC345 (manufactured by Dow Corning Toray Co., Ltd.), and KF-995 (manufactured by Shin-Etsu Chemical Co., Ltd.). Examples of commercial products the methyl trimethicone include silicone TMF-1.5 (manufactured by Shin-Etsu Chemical Co., Ltd.). Examples of commercial products of the decamethyltetrasiloxane include KF-96L-1.5CS (manufactured by Shin-Etsu Chemical Co., Ltd.). Examples of commercial products of the ethyl trisiloxane include SILSOFTETS (manufactured by Momentive Performance Materials Japan LLC.). Examples of commercial products of the volatile methyl polysiloxane include KF-96L-2CS (manufactured by Shin-Etsu Chemical Co., Ltd.).

Examples of the non-volatile oil agent include non-volatile hydrocarbon oils such as light liquid paraffin, liquid paraffin, heavy liquid isoparaffin, and squalane; non-volatile silicone oils such as non-volatile methyl polysiloxane and non-volatile methylphenyl polysiloxane; and non-volatile ester oils.

Examples of the non-volatile ester oil include non-volatile fatty acid ester oils such as cetyl 2-ethylhexanoate, isononyl isononanoate, isotridecyl isononanoate, isopropyl myristate, isopropyl palmitate, 2-ethylhexyl palmitate, 2-ethylhexyl stearate, and stearyl stearate; diisopropyl sebacate; and c12-15 alkyl benzoate. As the non-volatile fatty acid ester oil, fatty acid triglycerides such as caprylic/capric triglyceride and glyceryl tri(2-ethylhexanoate); esters of a fatty acid and neopentyl glycol such as neopentyl glycol dicaprate and neopentyl glycol diethylhexanoate; other polyhydric alcohol fatty acid ester oils, and the like may be used.

Among the non-volatile oil agents, non-volatile hydrocarbon oils and non-volatile ester oils are preferable.

Examples of commercial products of the liquid paraffin include ParLeam 4 (manufactured by NOF CORPORATION). Examples of commercial products of the non-volatile methyl polysiloxane include KF-96A-6CS (manufactured by Shin-Etsu Chemical Co., Ltd.). Examples of commercial products of the isopropyl palmitate include EXCEPARL IPP (manufactured by Kao Corporation). Examples of commercial products of the C12-15 alkyl benzoate include FINSOLV TN (manufactured by Innospec Active Chemicals). Examples of commercial products of the neopentyl glycol dicaprate include ESTEMOL N-01 (manufactured by the Nisshin OilliO Group, Ltd.).

The content of other liquid oil agents is preferably 0% by mass or more, more preferably 1% by mass or more, and further more preferably 5% by mass or more in the oil-in-water type cosmetic composition of the present invention, from the viewpoint of temporal stability and the like, and is preferably 25% by mass or less, more preferably 20% by mass or less, and further more preferably 15% by mass or less in the oil-in-water type cosmetic composition of the present invention, from the viewpoint of use impression and the like. The specific range is preferably 0% by mass or more and 25% by mass or less, more preferably 1% by mass or more and 20% by mass or less, and further preferably 5% by mass or more and 15% by mass or less in the oil-in-water type cosmetic composition of the present invention.

The oil-in-water type cosmetic composition of the present invention may comprise or may not comprise various polarity oils, and the total content of the polarity oil being liquid at 1 atm and 25°C is preferably 0% by mass or more and 40% by mass or less, more preferably 0% by mass or more and 30% by mass or less, and further preferably 0% by mass or more and 20% by mass or less, from the viewpoint of the ultraviolet protection effect and the like. For example, the aforementioned 2-ethylhexyl-p-methoxycinnamate and the like are polarity oils being liquid at 1 atm and 25°C, and the content of the 2-ethylhexyl-p-methoxycinnamate is preferably 0% by mass or more and 5% by mass or less, and more preferably 0% by mass or more and 3% by mass or less, and further preferably 0% by mass or more and 2% by mass or less, from the viewpoint of the ultraviolet protection effect and the like.

<Component (H)>

As the surfactant, known surfactants such as anionic surfactants, cationic surfactants, nonionic surfactants, and amphoteric surfactants may be used, but amphoteric surfactants (e.g., lecithin, hydrogenated lecithin, and lysolecithin) and nonionic surfactants are preferable.

Examples of the nonionic surfactant include polyhydric alcohol fatty acid ester type surfactants, polyoxyethylene fatty acid ester type surfactants, polyether-modified silicone type surfactants, polyoxyethylene sorbitan fatty acid ester type surfactants (e.g., polysorbate 20, polysorbate 60, polysorbate 65, and polysorbate 80), polyoxyethylene sorbitol fatty acid ester type surfactants, and polyoxyethylene alkyl ether type surfactants (e.g., Laureth-7). Examples of the polyhydric alcohol fatty acid ester type surfactant include glycerin fatty acid ester type surfactants, sorbitan fatty acid ester type surfactants (e.g., monosorbitan isostearate and monosorbitan oleate), diglycerin fatty acid ester type surfactants, polyglycerin fatty acid ester type surfactants, propylene glycol fatty acid ester type surfactants.

The surfactant may be used alone or in combination of two or more thereof.

The content of the surfactant is preferably 0% by mass or more, more preferably 0.1% by mass or more, further more preferably 0.2% by mass or more in the oil-in-water type cosmetic composition of the present invention, from the viewpoint of temporal stability and the like, and is preferably 15% by mass or less, more preferably 10% by mass or less, and further more preferably 5% by mass or less in the oil-in-water type cosmetic composition of the present invention, from the viewpoint of temporal stability, use impression, and the like. The specific range is preferably 0% by mass or more and 15% by mass or less, more preferably 0.1% by mass or more and 10% by mass or less, and further preferably 0.2% by mass or more and 5% by mass or less in the oil-in-water type cosmetic composition of the present invention.

The oil-in-water type cosmetic composition of the present invention may comprise a film forming agent such as (meth)acrylic silicone resins; a higher alcohol; a lower alcohol such as ethanol and propanol; a polyhydric alcohol such as ethylene glycol, propylene glycol, 1,3-butylene glycol, glycerin, diglycerin, and polyglycerin; a powder other than the component (D) (e.g., those mentioned in the component (D2) as the organic powder, inorganic powder, and organic-inorganic composite hydrophobized powder); a wax; a water-soluble polymer other than the component (C); a viscosity modifier; a sequestering agent; a pH regulator; a fragrance; a preservative; a conditioning agent; a wetting agent; a moisturizing agent; a refrigerant, and the like, in addition to the above components. These may be used alone or in combination of two or more thereof.

Examples of the dosage form of the oil-in-water type cosmetic composition of the present invention include non-solid forms such as a liquid form, an emulsion form, a cream form, a paste form, and a gel form, among which a liquid form, an emulsion form, and a cream form are preferable.

The oil-in-water type cosmetic composition of the present invention can be prepared as, for example, a lotion, an emulsion, a serum, a cream, and a foundation. The oil-in-water type cosmetic composition of the present invention is excellent in the ultraviolet protection effect, and thus is particularly suitable as a sunscreen cosmetic. The method of use thereof is not particularly limited, but is preferably applied with a hand or an applicator.

The oil-in-water type cosmetic composition of the present invention is suitably used as a skincare cosmetic, more suitably used by application to a skin except a scalp, and further suitably used by application to one or more selected from the group consisting of a face, a body, hands and foots.

The oil-in-water type cosmetic composition of the present invention can be produced in accordance with a conventional method.

The oil-in-water type cosmetic composition of the present invention is excellent in the ultraviolet protection effect and the effect of suppressing the shine caused by sebum, has excellent temporal stability, is unlikely to cause a reduction in temporal viscosity, and is unlikely to cause dripping after storage. It also has less stickiness after application. Though the reason why the excellent ultraviolet protection effect and the excellent effect of suppressing the shine caused by sebum can be imparted is not necessarily evident, the present inventors presume that the effect of suppressing the shine caused by sebum is improved by the presence of the component (B), and further, the reduction in the ultraviolet protection effect which tends to occur in the presence of the component (B) can be suppressed by incorporating the component (A) such that the mass ratio of [(B)/(A)] is 0.2 or more and 10 or less, and therefore the excellent ultraviolet protection effect can be imparted.

In relation to the aforementioned embodiments, the present invention further discloses the following oil-in-water type cosmetic compositions and the like.

<1> An oil-in-water type cosmetic composition comprising the following components (A), (B), (C), and (D) :

(A) an ultraviolet absorber having an intramolecular siloxane bond;
(B) a highly polymerized silicone oil;

(C) a poly(meth)acrylamide compound; and

(D) a hydrophobized powder,

wherein a mass ratio of the component (B) to the component (A), [(B)/(A)], is 0.2 or more and 10 or less.

<2> The oil-in-water type cosmetic composition according to <1>, wherein the component (A) is preferably one or more ultraviolet absorbers selected from the group consisting of a silicone-based ultraviolet absorber and an ultraviolet absorber having an intramolecular trisiloxane residue, more preferably one or more ultraviolet absorbers selected from the group consisting of a compound in which a part of or all methyl groups in dimethicone are substituted with diethyl 4-propynyloxybenzalmalonate, and an ultraviolet absorber having a trisiloxane residue and having a benzotriazole ring or a cinnamic acid ester skeleton in the molecule, further more preferably one or more selected from the group consisting of polysilicone-15 (dimethicodiethylbenzalmalonate), drometrizole trisiloxane, and isopentyl trimethoxycinnamate trisiloxane, and particularly preferably one or more selected from the group consisting of polysilicone-15 and drometrizole trisiloxane.

<3> The oil-in-water type cosmetic composition according to <1> or <2>, wherein a content of the component (A) is preferably 0.1% by mass or more, more preferably 0.5% by mass or more, further more preferably 0.7% by mass or more, further more preferably 1% by mass or more, and particularly preferably 1.3% by mass or more in the oil-in-water type cosmetic composition, and is preferably 15% by mass or less, more preferably 10% by mass or less, further more preferably 5% by mass or less, and particularly preferably 4% by mass or less in the oil-in-water type cosmetic composition.

<4> The oil-in-water type cosmetic composition according to <1> or <2>, wherein a content of the component (A) is preferably 0.1% by mass or more and 15% by mass or less, more preferably 0.5% by mass or more and 10% by mass or less, further more preferably 0.7% by mass or more and 5% by mass or less, further more preferably 1% by mass or more and 4% by mass or less, and particularly preferably 1.3% by mass or more and 4% by mass or less in the oil-in-water type cosmetic composition.

<5> The oil-in-water type cosmetic composition according to any one of <1> to <4>, wherein the component (B) is preferably a linear highly polymerized silicone oil being non-volatile liquid at 1 atm and 25°C.

<6> The oil-in-water type cosmetic composition according to any one of <1> to <5>, wherein a kinematic viscosity at 25°C of the component (B) is preferably 8 mm$^2$/s or more, more preferably 10 mm$^2$/s or more, further more preferably 15 mm$^2$/s or more, further more preferably 20 mm$^2$/s or more, and particularly preferably 50 mm$^2$/s or more, and is preferably 20 000 mm$^2$/s or less, more preferably 10 000 mm$^2$/s or less, further more preferably 7 500 mm$^2$/s or less, further more preferably 3 000 mm$^2$/s or less, further more preferably 1 000 mm$^2$/s or less, and particularly preferably 300 mm$^2$/s or less.

<7> The oil-in-water type cosmetic composition according to any one of <1> to <5>, wherein a kinematic viscosity at 25°C of the component (B) is preferably 8 mm$^2$/s or more and 10 000 mm$^2$/s or less, more preferably 10 mm$^2$/s or more and 7 500 mm$^2$/s or less, further more preferably 15 mm$^2$/s or more and 3 000 mm$^2$/s or less, further more preferably 20 mm$^2$/s or more and 1 000 mm$^2$/s or less, and particularly preferably 50 mm$^2$/s or more and 300 mm$^2$/s or less.

<8> The oil-in-water type cosmetic composition according to any one of <1> to <7>, wherein the component (B) is preferably one or more selected from the group consisting of dimethicone, methylphenyl polysiloxane, and methylhydrogenpolysiloxane, and more preferably dimethicone.

<9> The oil-in-water type cosmetic composition according to any one of <1> to <8>, wherein the content of the component (B) is preferably 0.1% by mass or more, more preferably 0.2% by mass or more, further more preferably 0.3% by mass or more, further more preferably 1.3% by mass or more, and particularly preferably 1.5% by mass or more in the oil-in-water type cosmetic composition, and is preferably 15% by mass or less, more preferably 10% by mass or less, further more preferably 7% by mass or less, and particularly preferably 5% by mass or less in the oil-in-water type cosmetic composition.

<10> The oil-in-water type cosmetic composition according to any one of <1> to <8>, wherein the content of the component (B) is preferably 0.1% by mass or more and 15% by mass or less, more preferably 0.3% by mass or more and 10% by mass or less, further more preferably 1.3% by mass or more and 7% by mass or less, and particularly preferably 1.5% by mass or more and 5% by mass or less in the oil-in-water type cosmetic composition.

<11> The oil-in-water type cosmetic composition according to any one of <1> to <10>, wherein a mass ratio of the component (B) to the component (A), [(B)/(A)], is preferably 0.4 or more, and more preferably 0.6 or more, and is preferably 5 or less, more preferably 3 or less, and particularly preferably 2 or less.

<12> The oil-in-water type cosmetic composition according to any one of <1> to <10>, wherein a mass ratio of the component (B) to the component (A), [(B)/(A)], is preferably 0.4 or more and 5 or less, more preferably 0.4 or more and 3 or less, further more preferably 0.6 or more and 3 or less, and particularly preferably 0.6 or more and 2 or less.

<13> The oil-in-water type cosmetic composition according to any one of <1> to <12>, wherein the component (C) is preferably a polymer having a repeating unit derived from (meth)acrylamide or a derivative thereof, more preferably one or more polymers selected from the group consisting of polyacrylamide, polymethacrylamide, a poly(acrylamide derivative), a poly(methacrylamide derivative), a copolymer of (meth)acrylamide and a (meth)acrylamide derivative, and a copolymer of one or two or more monomers selected from the group consisting of acrylamide, methacrylamide, an acrylamide derivative, and a methacrylamide derivative and other monomers, further more preferably a polymer having one or more repeating units selected from the group consisting of a repeating unit derived from (meth)acrylamide and a repeating unit derived from (meth)acryloyldimethyltaurine or a salt thereof, further more preferably one or more polymers selected from the group consisting of poly(meth)acrylamide, a copolymer of hydroxyethyl (meth)acrylate and a (meth)acryloyldimethyltaurine salt, a copolymer of a (meth)acrylic acid salt and a (meth)acryloyldimethyltaurine salt, a copolymer of (meth)acrylamide and a (meth)acrylic acid salt, a copolymer of (meth)acrylic acid, (meth)acrylamide, a (meth)acrylic acid salt, and a (meth)acryloyldimethyltaurine salt, and a copolymer of a (meth)acryloyldimethyltaurine salt and vinyl pyrrolidone, and particularly preferably one or more polymers selected from the group consisting of poly(meth)acrylamide, a copolymer of hydroxyethyl (meth)acrylate and a (meth)acryloyldimethyltaurine salt, a copolymer of a (meth)acrylic acid salt and a (meth)acryloyldimethyltaurine salt, and a copolymer of a (meth)acryloyldimethyltaurine salt and vinyl pyrrolidone.

<14> The oil-in-water type cosmetic composition according to any one of <1> to <13>, wherein a content of the component (C) is preferably 0.01% by mass or more, more preferably 0.05% by mass or more, further more preferably 0.1% by mass or more, and particularly preferably 0.3% by mass or more in the oil-in-water type cosmetic composition, and is preferably 7.5% by mass or less, more preferably 5% by mass or less, further more preferably 2.5% by mass or less, and particularly preferably 2% by mass or less in the oil-in-water type cosmetic composition.

<15> The oil-in-water type cosmetic composition according to any one of <1> to <13>, wherein a content of the component (C) is preferably 0.01% by mass or more and 7.5% by mass or less, more preferably 0.05% by mass or more and 5% by mass or less, further more preferably 0.1% by mass or more and 2.5% by mass or less, and particularly preferably 0.3% by mass or more and 2% by mass or less in the oil-in-water type cosmetic composition.

<16> The oil-in-water type cosmetic composition according to any one of <1> to <15>, wherein a mass ratio of the component (A) to the component (C), [(A)/(C)] is preferably 0.1 or more, more preferably 0.5 or more, further more preferably 1 or more, and particularly preferably 2 or more, and is preferably 20 or less, more preferably 15 or less, further more preferably 10 or less, and particularly preferably 5 or less.

<17> The oil-in-water type cosmetic composition according to any one of <1> to <15>, wherein a mass ratio of the component (A) to the component (C), [(A)/(C)] is preferably 0.1 or more and 20 or less, more preferably 0.5 or more and 15 or less, further more preferably 1 or more and 10 or less, and particularly preferably 2 or more and 5 or less.

<18> The oil-in-water type cosmetic composition according to any one of <1> to <17>, wherein a mass ratio of the component (B) to the component (C), [(B)/(C)] is preferably 0.1 or more, more preferably 1 or more, further more preferably 1.5 or more, and particularly preferably 2 or more, and is preferably 20 or less, more preferably 15 or less, further more preferably 10 or less, and particularly preferably 7.5 or less.

<19> The oil-in-water type cosmetic composition according to any one of <1> to <17>, wherein a mass ratio of the component (B) to the component (C), [(B)/(C)] is preferably 0.1 or more and 20 or less, more preferably 1 or more and 15 or less, and further more preferably 1.5 or more and 10 or less, and particularly preferably 2 or more and 7.5 or less.

<20> The oil-in-water type cosmetic composition according to any one of <1> to <19>, wherein the component (D) is preferably one or more selected from the group consisting of (D1) a hydrophobized ultraviolet scattering agent and (D2) a hydrophobized powder other than the hydrophobized ultraviolet scattering agent.

<21> The oil-in-water type cosmetic composition according to <20>, wherein the component (D1) is preferably a hydrophobized particulate metal oxide.

<22> The oil-in-water type cosmetic composition according to <21>, wherein the particulate metal oxide is preferably one or more particulate metal oxides selected from the group consisting of zinc oxide, titanium oxide, cerium oxide, iron oxide, and chromium oxide, more preferably one or more particulate metal oxides selected from the group consisting of zinc oxide, titanium oxide, and cerium oxide, and further more preferably one or more particulate metal oxides selected from the group consisting of zinc oxide and titanium oxide.

<23> The oil-in-water type cosmetic composition according to any one of <20> to <22>, wherein the component (D2) is preferably one or more selected from the group consisting of an organic hydrophobized powder, an inorganic hydrophobized powder, and an organic-inorganic composite hydrophobized powder, more preferably a powder obtained by subjecting one or more powder selected from the group consisting of a metal oxide having an average primary particle size $d_A$ of more than 100 nm, a pigment, a lake pigment, a polymer, an extender pigment, a pearl pigment, a metal salt, an inorganic powder, bentonite, smectite, boron nitride, and lauroyllysine to hydrophobization treatment.

<24> The oil-in-water type cosmetic composition according to any one of <20> to <23>, wherein a content of the component (D1) is preferably 1% by mass or more, more preferably 2% by mass or more, and further more preferably 4% by mass or more in the oil-in-water type cosmetic composition, and is preferably 25% by mass or less, more preferably 20% by mass or less, and further more preferably 15% by mass or less in the oil-in-water type cosmetic composition.

<25> The oil-in-water type cosmetic composition according to any one of <20> to <24>, wherein a content of the component (D2) is preferably 0% by mass or more, and more preferably 0.5% by mass or more in the oil-in-water type cosmetic composition, and is preferably 12% by mass or less, more preferably 8% by mass or less in the oil-in-water type cosmetic composition.

<26> The oil-in-water type cosmetic composition according to any one of <1> to <25>, wherein a content of the component (D) is preferably 1% by mass or more, more preferably 2% by mass or more, further more preferably 3% by mass or more, and particularly preferably 5% by mass or more in the oil-in-water type cosmetic composition, and is preferably 35% by mass or less, more preferably 30% by mass or less, further more preferably 20% by mass or less, and particularly preferably 17% by mass or less in the oil-in-water type cosmetic composition.

<27> The oil-in-water type cosmetic composition according to any one of <1> to <26>, wherein the content of the component (D) is preferably 1% by mass or more and 35% by mass or less, more preferably 2% by mass or more and 30% by mass or less, further more preferably 3% by mass or more and 20% by mass or less, and particularly preferably 5% by mass or more and 17% by mass or less in the oil-in-water type cosmetic composition.

<28> The oil-in-water type cosmetic composition according to any one of <1> to <27>, wherein a mass ratio of the component (D) to the component (B), [(D)/(B)], is preferably 0.1 or more, more preferably 0.5 or more, further more preferably 1 or more, and particularly preferably 2 or more, and is preferably 20 or less, more preferably 15 or less, further more preferably 13 or less, and particularly preferably 10 or less.

<29> The oil-in-water type cosmetic composition according to any one of <1> to <27>, wherein the mass ratio of the component (D) to the component (B), [(D)/(B)], is preferably 0.1 or more and 20 or less, more preferably 0.5 or more and 15 or less, further more preferably 1 or more and 13 or less, and particularly preferably 2 or more and 10 or less.

<30> The oil-in-water type cosmetic composition according to any one of <1> to <29>, wherein a content of (E) water is preferably 15% by mass or more, more preferably 20% by mass or more, further more preferably 25% by mass or more, and particularly preferably 30% by mass or more in the oil-in-water type cosmetic composition, and is preferably 80% by mass or less, more preferably 70% by mass or less, further more preferably 65% by mass or less, and particularly preferably 60% by mass or less in the oil-in-water type cosmetic composition.

<31> The oil-in-water type cosmetic composition according to any one of <1> to <29>, wherein a content of (E) water is preferably 15% by mass or more and 80% by mass or less, more preferably 20% by mass or more and 70% by mass or less, further more preferably 25% by mass or more and 65% by mass or less, and particularly preferably 30% by mass or more and 60% by mass or less in the oil-in-water type cosmetic composition.

<32> The oil-in-water type cosmetic composition according to any one of <1> to <31>, further comprising one or more selected from the group consisting of (F) an ultraviolet absorber other than the component (A), (G) a liquid oil agent, and (H) a surfactant.

<33> The oil-in-water type cosmetic composition according to any one of <1> to <32>, wherein a total content of a polarity oil being liquid at 1 atm and 25°C is preferably 0% by mass or more and 40% by mass or less, more preferably 0% by mass or more and 30% by mass or less, and further more preferably 0% by mass or more and 20% by mass or less.

<34> The oil-in-water type cosmetic composition according to any one of <1> to <33>, wherein the oil-in-water type cosmetic composition is preferably a liquid form, an emulsion form, a cream form, a paste form, or a gel form, and more preferably a liquid form, an emulsion form, or a cream form.

<35> The oil-in-water type cosmetic composition according to any one of <1> to <34>, wherein the oil-in-water type cosmetic composition is preferably a sunscreen cosmetic.

<36> An oil-in-water type cosmetic composition comprising the following components (A), (B), (C), and (D):

> (A) an ultraviolet absorber having an intramolecular siloxane bond: 0.1% by mass or more and 15% by mass or less;
> (B) a highly polymerized silicone oil: 1.3% by mass or more and 7% by mass or less;
> (C) a poly(meth)acrylamide compound: 0.1% by mass or more and 2.5% by mass or less; and
> (D) a hydrophobized powder: 3% by mass or more and 20% by mass or less,

wherein a mass ratio of the component (B) to the component (A), [(B)/(A)], is 0.2 or more and 10 or less.

<37> The oil-in-water type cosmetic composition according to <36>, wherein the component (A) is preferably one or more ultraviolet absorbers selected from the group consisting of a silicone-based ultraviolet absorber and an ultraviolet absorber having an intramolecular trisiloxane residue.

<38> The oil-in-water type cosmetic composition according to <36> or <37>, wherein a kinematic viscosity at 25°C of the component (B) is preferably 10 $mm^2$/s or more and 1 000 $mm^2$/s or less.

<39> The oil-in-water type cosmetic composition according to any one of <36> to <38>, wherein the component (C) is preferably a polymer having one or more repeating units selected from the group consisting of a repeating unit derived from (meth)acrylamide and a repeating unit derived from (meth)acryloyldimethyltaurine or a salt thereof.

<40> The oil-in-water type cosmetic composition according to any one of <36> to <39>, wherein a total content of a polarity oil being liquid at 1 atm and 25°C is preferably 0% by mass or more and 20% by mass or less.

<41> The oil-in-water type cosmetic composition according to any one of <36> to <40>, wherein a content of 2-ethylhexyl-p-methoxycinnamate is 0% by mass or more and 2% by mass or less.

Examples

Hereinafter, the present invention will be described in detail with reference to Examples, but the present invention is not limited to these Examples. Note that, various measurements and evaluations were carried out by the following methods in the examples.

Evaluation method

(1) Ultraviolet protection effect (SPF)

In accordance with the method described in paragraphs [0164] to [0165] of JP-A-2017-146211, an estimated SPF was calculated under the following conditions, and the ultraviolet protection effect was evaluated.

That is, a UV spectral image (center wavelength: 350 nm, half-width: 10 nm) of a test area was photographed in a darkroom, and then 2 mg/cm$^2$ of each test sample (cosmetic) was applied to the test area. The test area was dried for 15 minutes after application, and a UV spectral image was photographed again. Then, a transmittance T ($\lambda$) was determined using the equation (1) described in paragraph [0016] of JP-A-2017-146211, and thereafter, the estimated SPF (in vitro SPF) was calculated using the equation (2) described in paragraph [0047] of the same publication. Each parameter was the same as paragraph [0165] of JP-A-2017-146211. The evaluation criterion for the ultraviolet protection effect is shown below.

(Evaluation criterion for ultraviolet protection effect)

A: Estimated SPF: 50 or more
B: Estimated SPF: 35 or more and less than 50
C: Estimated SPF: less than 35

(2) Effect of suppressing the shine caused by sebum

A series of procedures in which each cosmetic was applied to the face after washing the face and then the absence of sebum shininess on the skin after the lapse of 6 hours from application was evaluated, was performed by ten female panelists. The evaluation criterion for the effect of suppressing sebum shininess is shown below.

(Evaluation criterion for effect of suppressing sebum shininess)

A: eight or more panelists answered that sebum shininess was felt to be suppressed.
B: three to seven panelists answered that sebum shininess was felt to be suppressed.
C: two or less panelists answered that sebum shininess was felt to be suppressed.

(3) Less stickiness after application

Sensory evaluation was carried out for less stickiness after application by ten professional panelists. The evaluation criterion for less stickiness after application is shown below. In addition to less stickiness after application, less friction feeling during application was evaluated. For the cosmetics of Examples 1 to 15 and 17 to 18, no panelists felt friction feeling in the sensory evaluation, but for the cosmetic of Example 16, five panelists felt friction feeling in the sensory evaluation.

(Evaluation criterion for less stickiness after application)

A: eight or more panelists answered that it is less sticky.
B: three or more and seven or less panelists answered that it is less sticky.
C: two or less panelists answered that it is less sticky.

(4) Temporal stability

The viscosity at 25°C of each cosmetic was measured under the following conditions. Then, each cosmetic was stored at 50°C for 1 month, and the viscosity at 25°C thereof was measured under the following conditions again. The viscosity after storage was subtracted from the viscosity before storage, and the temporal stability was evaluated based on the obtained viscosity reduction range. The viscosity before storage of the cosmetic of Example 1 was 170 000 mPa·s, and the viscosity before storage of the cosmetic of Comparative Example 4 was 125 000 mPa·s.

(Viscosity measurement conditions)

Apparatus: TVB-15 manufactured by Toki Sangyo Co., Ltd
Roter: No.4
Rotational speed: 3 rpm
Measurement time: 1 minute

(Evaluation criterion for temporal stability)

Good: viscosity reduction range was 70 000 mPa·s or less
Fair: viscosity reduction range was more than 70 000 mPa·s and 85 000 mPa·s or less
Poor: viscosity reduction range was more than 85 000 mPa·s

(Examples 1 to 18 and Comparative Examples 1 to 7: oil-in-water type cosmetic composition)

The oil-in-water type cosmetic compositions of Examples 1 to 18 and Comparative Examples 1 to 4 and 7 were produced by an ordinary method in accordance with the formulations shown in Tables 1 to 3, and the ultraviolet protection effect (SPF), the effect of suppressing the shine caused by sebum, less stickiness after application, and temporal stability were evaluated. The results are shown in Tables 1 to 3.

For the formulations of Comparative Examples 5 to 6 shown in Table 3, the viscosity was increased during production, which made stirring impossible. Thus the production type cosmetic compositionwas not successful anyway.

[Table 1]

| Component (% by mass) | | Example | | | | | | | | |
|---|---|---|---|---|---|---|---|---|---|---|
| | | 1 | 2 | 3 | 4 | 5 | 6 | 7 | 8 | 9 |
| (A) | Polysilicone-15 | 2 | 10 | 0.5 | | 1.5 | 2 | 2 | 2 | 2 |
| | Drometrizole trisiloxane | | | | 3 | 1.5 | | | | |
| Ethylhexyl triazone | | 3 | 3 | 3 | 3 | 3 | 3 | 3 | 3 | 3 |
| Bis-ethylhexyloxyphenol methoxyphenyl triazine | | 1 | 1 | 1 | 1 | 1 | 1 | 1 | 1 | 1 |
| Diethylamino hydroxybenzoyl hexyl benzoate | | 0.5 | 0.5 | 0.5 | 0.5 | 0.5 | 0.5 | 0.5 | 0.5 | 0.5 |
| (B) | Dimethicone (kinematic viscosity: 100 mm$^2$/s) *1 | 2 | 2 | 2 | 2 | 2 | | | | |
| | Dimethicone (kinematic viscosity: 10 mm$^2$/s) *2 | | | | | | 2 | | | |
| | Dimethicone (kinematic viscosity: 50 mm$^2$/s) *3 | | | | | | | 2 | | |
| | Dimethicone (kinematic viscosity: 5000 mm$^2$/s) *4 | | | | | | | | 2 | |
| | Dimethicone (kinematic viscosity: 10 000 mm$^2$/s) *5 | | | | | | | | | 2 |
| Dimethicone (kinematic viscosity: 6 mm$^2$/s) *6 | | | | | | | | | | |
| (C) | SIMULGEL EG (manufactured by SEPPIC) *7 | 2 | 2 | 2 | 2 | 2 | 2 | 2 | 2 | 2 |
| | SEPIGEL 305 (manufactured by SEPPIC) *8 | | | | | | | | | |
| | SEPINOV EMT 10 (manufactured by SEPPIC) *9 | | | | | | | | | |
| | (Acryloyldimethyltaurine ammonium/vinylpyrrolidone) copolymer | | | | | | | | | |
| Carbomer | | | | | | | | | | |

| Component (% by mass) | | Example | | | | | | | | |
|---|---|---|---|---|---|---|---|---|---|---|
| | | 1 | 2 | 3 | 4 | 5 | 6 | 7 | 8 | 9 |
| (D2) | ASI-treated talc | 1 | | | | | | | | |
| | ASI-treated red iron oxide | 0.1 | | | | | | | | |
| | Red No. 226 | 0.1 | | | | | | | | |
| | Ultramarine | 0.1 | | | | | | | | |
| (D2) | Hydrophobized titanium oxide *10 | 3 | 2 | 2 | 2 | 2 | 2 | 2 | 2 | 2 |
| (D1) | Hydrophobized particulate titanium oxide *11 | 5 | 5 | 5 | 5 | 5 | 5 | 5 | 5 | 5 |
| | Hydrophobized particulate zinc oxide *12 | 5 | 5 | 5 | 5 | 5 | 5 | 5 | 5 | 5 |
| Diisopropyl sebacate | | 7 | 7 | 7 | 7 | 7 | 7 | 7 | 7 | 7 |
| Triethylhexanoin | | 4 | 3 | 3 | 3 | 3 | 3 | 3 | 3 | 3 |
| Alkyl benzoate | | | 1 | 1 | 1 | 1 | 1 | 1 | 1 | 1 |
| DOWSIL FA 4003 DM SILICONE ACRYLATE (manufactured by Dow Corning Toray Co., Ltd.) *13 | | 1 | | | | | | | | |
| Hydrogenated lecithin | | 1 | 1 | 1 | 1 | 1 | 1 | 1 | 1 | 1 |
| Behenyl alcohol | | 1 | 1 | 1 | 1 | 1 | 1 | 1 | 1 | 1 |
| Glycerin | | 3 | 3 | 3 | 3 | 3 | 3 | 3 | 3 | 3 |
| 1,3-Butylene glycol | | 2 | 2 | 2 | 2 | 2 | 2 | 2 | 2 | 2 |
| Xanthan gum | | 0.1 | 0.1 | 0.1 | 0.1 | 0.1 | 0.1 | 0.1 | 0.1 | 0.1 |
| Disodium edetate | | 0.02 | 0.02 | 0.02 | 0.02 | 0.02 | 0.02 | 0.02 | 0.02 | 0.02 |
| Ethanol | | 10 | 10 | 10 | 10 | 10 | 10 | 10 | 10 | 10 |
| Potassium hydroxide | | | | | | | | | | |
| Purified water | | balance | balance | balance | balance | balance | balance | balance | balance | balance |
| Total | | 100 | 100 | 100 | 100 | 100 | 100 | 100 | 100 | 100 |
| Mass ratio [(B)/(A)] | | 1.0 | 0.2 | 4.0 | 0.7 | 0.7 | 1.0 | 1.0 | 1.0 | 1.0 |

EP 4 248 941 A1

(continued)

| Component (% by mass) | | Example | | | | | | | | |
|---|---|---|---|---|---|---|---|---|---|---|
| | | 1 | 2 | 3 | 4 | 5 | 6 | 7 | 8 | 9 |
| Evaluation results | Ultraviolet protection effect (SPF) | A | A | B | A | A | A | A | A | A |
| | Effect of suppressing sebum shininess | A | A | B | A | A | A | A | A | A |
| | Less stickiness after application | A | B | A | A | A | A | A | B | B |
| | Temporal stability | Good | Good | Good | Good | Good | Good | Good | Good | Good |

[Table 2]

| Component (% by mass) | | Example | | | | | | | | |
|---|---|---|---|---|---|---|---|---|---|---|
| | | 10 | 11 | 12 | 13 | 14 | 15 | 16 | 17 | 18 |
| (A) | Polysilicone-15 | 2 | 2 | 2 | 2 | 2 | 2 | 2 | 2 | 2 |
| | Drometrizole trisiloxane | | | | | | | | | |
| Ethylhexyl triazone | | 3 | 3 | 3 | 3 | 3 | 3 | 3 | 3 | 3 |
| Bis-ethylhexyloxyphenol methoxyphenyl triazine | | 1 | 1 | 1 | 1 | 1 | 1 | 1 | 1 | 1 |
| Diethylamino hydroxybenzoyl hexyl benzoate | | 0.5 | 0.5 | 0.5 | 0.5 | 0.5 | 0.5 | 0.5 | 0.5 | 0.5 |
| (B) | Dimethicone (kinematic viscosity: 100 mm$^2$/s) *1 | 1 | 4 | 10 | 2 | 2 | 2 | 2 | 2 | 2 |
| | Dimethicone (kinematic viscosity: 10 mm$^2$/s) *2 | | | | | | | | | |
| | Dimethicone (kinematic viscosity: 50 mm$^2$/s) *3 | | | | | | | | | |
| | Dimethicone (kinematic viscosity: 5000 mm$^2$/s) *4 | | | | | | | | | |
| | Dimethicone (kinematic viscosity: 10 000 mm$^2$/s) *5 | | | | | | | | | |
| Dimethicone (kinematic viscosity:6 mm$^2$/s) *6 | | | | | | | | | | |
| (C) | SIMULGEL EG (manufactured by SEPPIC) *7 | 2 | 2 | 2 | | | | 2 | 2 | 2 |
| | SEPIGEL 305 (manufactured by SEPPIC) *8 | | | | 1.5 | | | | | |
| | SEPINOV EMT 10 (manufactured by SEPPIC) *9 | | | | | 2 | | | | |
| | (Acryloyldimethyltaurine ammonium/vinylpyrrolidone) copolymer | | | | | | 1 | | | |
| Carbomer | | | | | | | | | | |

EP 4 248 941 A1

(continued)

| Component (% by mass) | | Example | | | | | | | | |
|---|---|---|---|---|---|---|---|---|---|---|
| | | 10 | 11 | 12 | 13 | 14 | 15 | 16 | 17 | 18 |
| (D2) | ASI-treated talc | | | | | | | | | |
| | ASI-treated red iron oxide | | | | | | | | | |
| | Red No. 226 | | | | | | | | | |
| | Ultramarine | | | | | | | | | |
| (D2) | Hydrophobized titanium oxide *10 | 2 | 2 | 2 | 2 | 2 | 2 | 10 | | |
| (D1) | Hydrophobized particulate titanium oxide *11 | 5 | 5 | 5 | 5 | 5 | 5 | 10 | | |
| | Hydrophobized particulate zinc oxide *12 | 5 | 5 | 5 | 5 | 5 | 5 | 10 | 10 | 10 |
| Diisopropyl sebacate | | 7 | 7 | 7 | 7 | 7 | 7 | 7 | 7 | 7 |
| Triethylhexanoin | | 3 | 3 | 3 | 3 | 3 | 3 | 3 | 3 | 3 |
| Alkyl benzoate | | 1 | 1 | 1 | 1 | 1 | 1 | 1 | 1 | 1 |
| DOWSIL FA 4003 DM SILICONE ACRYLATE (manufactured by Dow Corning Toray Co., Ltd.) *13 | | | | | | | | | | |
| Hydrogenated lecithin | | 1 | 1 | 1 | 1 | 1 | 1 | 1 | 1 | |
| Behenyl alcohol | | 1 | 1 | 1 | 1 | 1 | 1 | 1 | 1 | |
| Glycerin | | 3 | 3 | 3 | 3 | 3 | 3 | 3 | 3 | 3 |
| 1,3-Butylene glycol | | 2 | 2 | 2 | 2 | 2 | 2 | 2 | 2 | 2 |
| Xanthan gum | | 0.1 | 0.1 | 0.1 | 0.1 | 0.1 | 0.1 | 0.1 | 0.1 | 0.1 |
| Disodium edetate | | 0.02 | 0.02 | 0.02 | 0.02 | 0.02 | 0.02 | 0.02 | 0.02 | 0.02 |
| Ethanol | | 10 | 10 | 10 | 10 | 10 | 10 | 10 | 10 | 10 |
| Potassium hydroxide | | | | | | | | | | |
| Purified water | | balance | balance | balance | balance | balance | balance | balance | balance | balance |
| Total | | 100 | 100 | 100 | 100 | 100 | 100 | 100 | 100 | 100 |
| Mass ratio [(B)/(A)] | | 0.5 | 2.0 | 5.0 | 1.0 | 1.0 | 1.0 | 1.0 | 1.0 | 1.0 |

| Component (% by mass) | | Example | | | | | | | | |
|---|---|---|---|---|---|---|---|---|---|---|
| | | 10 | 11 | 12 | 13 | 14 | 15 | 16 | 17 | 18 |
| Evaluation results | Ultraviolet protection effect (SPF) | A | A | B | A | A | A | A | A | A |
| | Effect of suppressing sebum shininess | B | A | A | A | A | A | A | A | A |
| | Less stickiness after application | A | A | B | A | A | A | B | A | A |
| | Temporal stability | Good | Good | Good | Good | Good | Good | Good | Good | Good |

[Table 3]

| Component (% by mass) | | Comparative Example 5 | | | | | | |
|---|---|---|---|---|---|---|---|---|
| | | 1 | 2 | 3 | 4 | 5 | 6 | 7 |
| (A) | Polysilicone-15 | | | 2 | 2 | 2 | 2 | 10 |
| | Drometrizole trisiloxane | | | | | | | |
| Ethylhexyl triazone | | 3 | 5 | 3 | 3 | 3 | 3 | 3 |
| Bis-ethylhexyloxyphenol methoxyphenyl triazine | | 1 | 1 | 1 | 1 | 1 | 1 | 1 |
| Diethylamino hydroxybenzoyl hexyl benzoate | | 0.5 | 0.5 | 0.5 | 0.5 | 0.5 | 0.5 | 0.5 |
| (B) | Dimethicone (kinematic viscosity:100mm$^2$/s) *1 | 2 | 2 | | 2 | 2 | 2 | 1 |
| | Dimethicone (kinematic viscosity:10mm$^2$/s) *2 | | | | | | | |
| | Dimethicone (kinematic viscosity: 50 mm$^2$/s) *3 | | | | | | | |
| | Dimethicone (kinematic viscosity: 5000 mm$^2$/s) *4 | | | | | | | |
| | Dimethicone (kinematic viscosity: 10 000 mm$^2$/s) *5 | | | | | | | |
| Dimethicone (kinematic viscosity:6mm$^2$/s) *6 | | | | 2 | | | | |
| (C) | SIMULGEL EG (manufactured by SEPPIC) *7 | 2 | 2 | 2 | | | 2 | 2 |
| | SEPIGEL 305 (manufactured by SEPPIC) *8 | | | | | | | |
| | SEPINOV EMT 10 (manufactured by SEPPIC) *9 | | | | | | | |
| | (Acryloyldimethyltaurine ammonium/ vinylpyrrolidone) copolymer | | | | | | | |
| Carbomer | | | | | 0.2 | 0.75 | | |
| (D2) | ASI-treated talc | | | | | | | |
| | ASI-treated red iron oxide | | | | | | | |
| | Red No. 226 | | | | | | | |
| | Ultramarine | | | | | | | |
| (D2) | Hydrophobized titanium oxide *10 | 2 | 2 | 2 | 2 | 2 | | 2 |

EP 4 248 941 A1

22

(continued)

| Component (% by mass) | | Comparative Example | | | | | | |
|---|---|---|---|---|---|---|---|---|
| | | 1 | 2 | 3 | 4 | 5 | 6 | 7 |
| (D1) | Hydrophobized particulate titanium oxide *11 | 5 | 5 | 5 | 5 | 5 | | 5 |
| | Hydrophobized particulate zinc oxide *12 | 5 | 5 | 5 | 5 | 5 | | 5 |
| | Hydrophilic particulate titanium oxide *14 | | | | | | 12 | |
| Diisopropyl sebacate | | 7 | 7 | 7 | 7 | 7 | 7 | 7 |
| Triethylhexanoin | | 3 | 3 | 3 | 3 | 3 | 3 | 3 |
| Alkyl benzoate | | 1 | 1 | 1 | 1 | 1 | 1 | 1 |
| DOWSIL FA 4003 DM SILICONE ACRYLATE (manufactured by Dow Corning Toray Co., Ltd.) *13 | | | | | | | | |
| Hydrogenated lecithin | | 1 | 1 | 1 | 1 | 1 | 1 | 1 |
| Behenyl alcohol | | 1 | 1 | 1 | 1 | 1 | 1 | 1 |
| Glycerin | | 3 | 3 | 3 | 3 | 3 | 3 | 3 |
| 1,3-Butylene glycol | | 2 | 2 | 2 | 2 | 2 | 2 | 2 |
| Xanthan gum | | 0.1 | 0.1 | 0.1 | 0.1 | 0.1 | 0.1 | 0.1 |
| Disodium edetate | | 0.02 | 0.02 | 0.02 | 0.02 | 0.02 | 0.02 | 0.02 |
| Ethanol | | 10 | 10 | 10 | 10 | 10 | 10 | 10 |
| Potassium hydroxide | | | | | 0.5 | 0.5 | | |
| Purified water | | balance | balance | balance | balance | balance | balance | balance |
| Total | | 100 | 100 | 100 | 100 | 100 | 100 | 100 |
| Mass ratio [(B)/(A)] | | - | - | 0.0 | 1.0 | 1.0 | 1.0 | 0.1 |
| Evaluation results | Ultraviolet protection effect (SPF) | C | A | A | - | production impossible | production impossible | A |
| | Effect of suppressing sebum shininess | C | C | C | - | | | B |
| | Less stickiness after application | A | B | B | - | | | C |
| | Temporal stability | Good | Good | Good | Poor | | | Good |

23

The symbols in the Tables represent the followings.

*1: KF-96A-100CS (manufactured by Shin-Etsu Chemical Co., Ltd.)
*2: KF-96A-10CS (manufactured by Shin-Etsu Chemical Co., Ltd.)
*3: KF-96A-50CS (manufactured by Shin-Etsu Chemical Co., Ltd.)
*4: KF-96A-5000CS (manufactured by Shin-Etsu Chemical Co., Ltd.)
*5: KF-96A-10000CS (manufactured by Shin-Etsu Chemical Co., Ltd.)
*6: KF-96A-6CS (manufactured by Shin-Etsu Chemical Co., Ltd.)
*7: A mixture of a sodium acrylate/sodium acryloyldimethyl taurate copolymer: 37.5% by mass, isohexadecane: 22.5% by mass, polysorbate 80: 7.5% by mass, sorbitan oleate: 2.5% by mass, and water: 30.0% by mass, for example, the content of the sodium acrylate/sodium acryloyldimethyl taurate copolymer in the cosmetic in Example 1 is 0.75% by mass.
*8: A mixture of polyacrylamide: 40% by mass, hydrogenated polyisobutene: 30% by mass, laureth-7: 24% by mass, and water: 6% by mass
*9: A mixture of a hydroxyethyl acrylate/sodium acryloyldimethyl taurate copolymer: 90% by mass, sorbitan isostearate: 5% by mass, and polysorbate 60: 5% by mass
*10: Silica/aluminum hydroxide/ASI-treated titanium oxide (average primary particle size: 270 nm)
*11: Silica/triethoxycaprylylsilane-treated particulate titanium oxide (average primary particle size: 15 nm)
*12: Dimethicone/triethoxycaprylylsilane-treated particulate zinc oxide (average primary particle size: 35 nm)
*13: A mixture of an acrylates/polytrimethylsiloxymethacrylate copolymer: 70% by mass and decamethylcyclopentasiloxane: 30% by mass
*14: SUNSIL Tin 50 (manufactured by SUNJIN CHEMICAL)

(Example 19: oil-in-water type cosmetic composition)

The oil-in-water type cosmetic composition of Example 19 was also excellent in the ultraviolet protection effect (SPF), the effect of suppressing the shine caused by sebum, less stickiness after application, and temporal stability.

[Table 4]

| Component (% by mass) | | Example 19 |
|---|---|---|
| (A) | Polysilicone-15 | 2 |
| | Drometrizole trisiloxane | |
| Ethylhexyl triazone | | 3 |
| Bis-ethylhexyloxyphenol methoxyphenyl triazine | | 1 |
| Diethylamino hydroxybenzoyl hexyl benzoate | | 0.5 |
| Ethylhexyl methoxycinnamate | | 2 |
| (B) | Dimethicone (kinematic viscosity: 100 mm$^2$/s) *1 | 2 |
| | Dimethicone (kinematic viscosity: 10 mm$^2$/s) *2 | |
| | Dimethicone (kinematic viscosity: 50 mm$^2$/s) *3 | |
| | Dimethicone (kinematic viscosity: 5 000 mm$^2$/s) *4 | |
| | Dimethicone (kinematic viscosity: 10 000 mm$^2$/s) *5 | |
| Dimethicone (kinematic viscosity:6 mm$^2$/s) *6 | | |
| (C) | SIMULGEL EG (manufactured by SEPPIC) *7 | 2 |
| | SEPIGEL 305 (manufactured by SEPPIC) *8 | |
| | SEPINOV EMT 10 (manufactured by SEPPIC) *9 | |
| | (Acryloyldimethyltaurine ammonium/vinylpyrrolidone) copolymer | |
| Carbomer | | |

(continued)

| Component (% by mass) | | Example 19 |
|---|---|---|
| (D2) | ASI-treated talc | 1 |
| | ASI-treated red iron oxide | 0.1 |
| | Red No. 226 | 0.1 |
| | Ultramarine | 0.1 |
| (D2) | Hydrophobized titanium oxide *10 | 3 |
| (D1) | Hydrophobized particulate titanium oxide *11 | 5 |
| | Hydrophobized particulate zinc oxide *12 | 5 |
| Diisopropyl sebacate | | 5 |
| Triethylhexanoin | | 4 |
| Alkyl benzoate | | |
| DOWSIL FA 4003 DM SILICONE ACRYLATE (manufactured by Dow Corning Toray Co., Ltd.) *13 | | 1 |
| Hydrogenated lecithin | | 1 |
| Behenyl alcohol | | 1 |
| Glycerin | | 3 |
| 1,3-Butylene glycol | | 2 |
| Xanthan gum | | 0.1 |
| Disodium edetate | | 0.02 |
| Ethanol | | 10 |
| Potassium hydroxide | | |
| Purified water | | balance |
| Total | | 100 |
| Mass ratio [(B)/(A)] | | 1.0 |

**Claims**

1. An oil-in-water type cosmetic composition comprising the following components (A), (B), (C), and (D):

   (A) an ultraviolet absorber having an intramolecular siloxane bond;
   (B) a highly polymerized silicone oil;
   (C) a poly(meth)acrylamide compound; and
   (D) a hydrophobized powder,

   wherein a mass ratio of the component (B) to the component (A), [(B)/(A)], is 0.2 or more and 10 or less.

2. The oil-in-water type cosmetic composition according to claim 1, wherein the component (A) is one or more ultraviolet absorbers selected from the group consisting of a silicone-based ultraviolet absorber and an ultraviolet absorber having an intramolecular trisiloxane residue.

3. The oil-in-water type cosmetic composition according to claim 1 or 2, wherein a content of the component (A) is 0.1% by mass or more and 15% by mass or less in the oil-in-water type cosmetic composition.

4. The oil-in-water type cosmetic composition according to any one of claims 1 to 3, wherein the content of the component (A) is 1% by mass or more and 4% by mass or less in the oil-in-water type cosmetic composition.

5. The oil-in-water type cosmetic composition according to any one of claims 1 to 4, wherein the component (B) is a highly polymerized silicone oil having a kinematic viscosity at 25°C of 8 mm$^2$/s or more and 10 000 mm$^2$/s or less.

6. The oil-in-water type cosmetic composition according to any one of claims 1 to 5, wherein the component (B) is a highly polymerized silicone oil having a kinematic viscosity at 25°C of 10 mm$^2$/s or more and 1 000 mm$^2$/s or less.

7. The oil-in-water type cosmetic composition according to any one of claims 1 to 6, wherein a content of the component (B) is 1.3% by mass or more and 7% by mass or less in the oil-in-water type cosmetic composition.

8. The oil-in-water type cosmetic composition according to any one of claims 1 to 7, wherein the component (C) is a polymer having one or more repeating units selected from the group consisting of a repeating unit derived from (meth)acrylamide and a repeating unit derived from (meth)acryloyldimethyltaurine or a salt thereof.

9. The oil-in-water type cosmetic composition according to any one of claims 1 to 8, wherein a content of the component (C) is 0.1% by mass or more and 2.5% by mass or less in the oil-in-water type cosmetic composition.

10. The oil-in-water type cosmetic composition according to any one of claims 1 to 9, wherein the component (D) comprises (D1) a hydrophobized ultraviolet scattering agent.

11. The oil-in-water type cosmetic composition according to any one of claims 1 to 10, wherein a content of the component (D) is 3% by mass or more and 20% by mass or less in the oil-in-water type cosmetic composition.

12. The oil-in-water type cosmetic composition according to any one of claims 1 to 11, wherein a total content of a polarity oil in a liquid state at 1 atm and 25°C is 0% by mass or more and 20% by mass or less.

13. The oil-in-water type cosmetic composition according to any one of claims 1 to 12, wherein a content of 2-ethylhexyl-p-methoxycinnamate is 0% by mass or more and 2% by mass or less.

14. The oil-in-water type cosmetic composition according to any one of claims 1 to 13, wherein the oil-in-water type cosmetic composition is a sunscreen cosmetic.

## INTERNATIONAL SEARCH REPORT

| International application No. |
|---|
| **PCT/JP2021/042525** |

**A.    CLASSIFICATION OF SUBJECT MATTER**

*A61K 8/58*(2006.01)i; *A61K 8/06*(2006.01)i; *A61K 8/88*(2006.01)i; *A61K 8/895*(2006.01)i; *A61Q 17/04*(2006.01)i
FI:    A61K8/58; A61K8/06; A61K8/88; A61K8/895; A61Q17/04

According to International Patent Classification (IPC) or to both national classification and IPC

**B.    FIELDS SEARCHED**

Minimum documentation searched (classification system followed by classification symbols)

A61K8/58; A61K8/06; A61K8/88; A61K8/895; A61Q17/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); Japio-GPG/FX

**C.    DOCUMENTS CONSIDERED TO BE RELEVANT**

| Category* | Citation of document, with indication, where appropriate, of the relevant passages | Relevant to claim No. |
|---|---|---|
| X | JP 2017-39664 A (L'OREAL) 23 February 2017 (2017-02-23)<br>claims, paragraphs [0001], [0010], [0032]-[0064], [0089]-[0123], [0166]-[0186], [0216], [0219] | 1-4, 8-10, 12, 14 |
| Y | | 5-7, 11, 13 |
| Y | JP 2011-016733 A (KAO CORP) 27 January 2011 (2011-01-27)<br>paragraph [0042] | 5-7, 11, 13 |
| A | JP 2017-155048 A (SHISEIDO CO LTD) 07 September 2017 (2017-09-07)<br>entire text | 1-14 |
| A | JP 2007-23031 A (L'OREAL) 01 February 2007 (2007-02-01)<br>entire text | 1-14 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **11 January 2022** | **01 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/042525**

| Patent document cited in search report | | | Publication date (day/month/year) | Patent family member(s) | | | Publication date (day/month/year) |
|---|---|---|---|---|---|---|---|
| JP | 2017-39664 | A | 23 February 2017 | US | 2018/0185253 | A1 | |
| | | | | claims, paragraphs [0001], [0008], [0037]-[0096], [0121]-[0153], [0189]-[0211], [0238], [0241] | | | |
| JP | 2011-016733 | A | 27 January 2011 | (Family: none) | | | |
| JP | 2017-155048 | A | 07 September 2017 | WO | 2017/056506 | A1 | |
| JP | 2007-23031 | A | 01 February 2007 | US | 2007/0009453 | A1 | |
| | | | | entire text | | | |
| | | | | FR | 2888112 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013136569 A
- JP H2212579 A
- JP H3220153 A
- JP 2017146211 A